# EUROPÄISCHE PATENTANMELDUNG

(11) **EP 1 033 140 A2**
(43) Veröffentlichungstag der Anmeldung: **06.09.2000**
(21) Anmeldenummer: 00104075.7
(22) Anmeldetag: 28.02.2000
(51) Int. Cl.: A61L 9/04, A61L 9/12, A61L 9/14, A01M 1/20, A45D 34/00, A01M 13/00

(54) **Verdunstung von Flüssigkeiten in einen Luftstrom**

(30) Priorität: 03.03.1999 DE 19909218
(71) Anmelder: Climarotec Gesellschaft für raumklimatische Spezialanlagen mbH, 61350 Bad Homburg (DE)
(72) Erfinder: Dierssen, Jens, Dr. Dipl.-Chem., 61350 Bad Homburg (DE)

(57) **Zusammenfassung**

Gegenstand der Erfindung ist ein Verfahren zur effizienten und steuerbaren gasförmigen Verdunstung von Flüssigkeiten in Raumluft mittels eines Luftstromes und eine Vorrichtung zur Durchführung des Verfahrens. Die Erfindung findet bevorzugt Anwendung bei der Ausbringung von Duftstoffen in Raumluft.

Die vorgestellte Vorrichtung und das Verfahren ermöglicht es mittels einer vorhandenen oder gesteuerten Luftströmung definierte Mengen Duftstoff aus einem umschlossenen Behälter freizusetzen. Ein Teilstrom eines Luftstromes durchläuft den Behälter und reichert sich dabei mit dem Duftstoff an. Der Behälter kann beliebig bewegt werden ohne Duftstoff zu verschütten.

## Beschreibung

### Technisches Gebiet

Gegenstand der Erfindung ist ein Verfahren zur effizienten Verdunstung von Flüssigkeiten in Raumluft durch einen vorhandenen Luftstrom sowie die dafür notwendigen Vorrichtungen.

Die Erfindung findet Anwendung auf allen technischen Gebieten, auf denen eine effiziente und technisch einfache Verdunstung von Flüssigkeiten in ein Luftvolumen notwendig ist. Insbesondere findet die vorliegende Erfindung Anwendung bei der Aromatisierung bzw. Beduftung von Räumen.
Desweiteren findet die Erfindung Anwendung in der Medizin, wie beispielsweise der Aromatherapie oder der Inhalationstherapie flüssiger Therapeutika. Der Gegenstand der Erfindung kann auch verwendet werden, um eine bakteriostatische Wirkung zu vermitteln wie sie zur Sterilisierung von Räumen notwendig ist.
Auch in der Landwirtschaft erweist sich der Gegenstand der Erfindung als vorteilhaft.
Unter anderem werden derzeit flüssige Lock- und Duftstoffe, sowie Schreck- und Hormonstoffe, Insektizide und Fungizide in der Landwirtschaft in definierten Konzentrationen in Gewächshäusern und freien Feldern ausgebracht.

### Stand der Technik

Bei der Darstellung des Standes der Technik wird beispielhaft auf die Verdunstung von Flüssigkeiten zur Aromatisierung bzw. Beduftung von Räumen eingegangen. Die Erfindung ist jedoch keineswegs auf eine solche Anwendung beschränkt.

In den letzten Jahren nimmt die Beduftung von Wohn- und Geschäftsräumen, Fahrstühlen, sanitären Anlagen, stark frequentierten Räumen, Fluren, Anlagen und selbst Fahrzeugen immer mehr an Bedeutung zu.
Zur Zeit stehen zur Vedunstung flüssiger Duftstoffe eine Reihe technischer Lösungen zur Verfügung. Neben der Verdampfung durch Erhitzen wird auch mittels Düsen die Flüssigkeit vernebelt wie bei Raumsprühern und Klimaanlagen derzeit üblich. Bei der Beduftung kleiner Raumvolumen genügt oft schon das einfache Verdunsten ohne Erhöhung der Flüssigkeitsoberfläche oder Erwärmung.
Duftbäume, Zerstäuber, Duftkerzen, Duftlampen und andere technische Vorrichtungen sind zur Beduftung üblich. Die dadurch ausgebrachte Flüssigkeitsmenge wird jedoch durch die Dimensionierung der Vorrichtung und die Dauer des Einschaltens des Geräts bestimmt und ermöglicht deshalb keine auf die Räumlichkeit abgestimmte definierte Verdampfung.
Typischerweise weisen im Sanitärbereich eingesetzte Duftsprüher eine lokale und kurzzeitig überhöhte Duftstoffkonzentration auf. Mit Duftstoffen getränkte bzw. imprägnierte Gegenstände setzen ihren Duftstoff dauerhaft frei, was beispielsweise im PKW schnell zu einer Überdosierung führt.
Im Bereich der Klimaanlagen werden Duftstoffe durch Versprühen und Verdampfen ausgebracht. Die geschieht jedoch mit erheblichem technischen Aufwand. Auch ist die Wartung und Reinigung solcher Vorrichtungen aufwendig.

Als der Erfindung nächster Stand der Technik können folgende 2 Verfahren angesehen werden.
Bei dem ersten Verfahren wird einfach eine offene Schale mit Duftflüssigkeit zur Verdunstung in einen Raum gestellt. Zur Verstärkung der Verdunstung kann die Flüssigkeit erhitzt werden (Kerze). Bei einer offenen Schale ist das mit Flüssigkeit beladene Gefäß ohne Verschütten zu bewegen. Zum An- und Ausschalten der Vorrichtung muß diese oder die Flüssigkeit entfernt werden. Eine andere Regelung ist nicht möglich.
Das zweite Verfahren ist ein mit Duftstoff angereichertes saugfähiges Stück
Flies oder Zellulose welches sich offen in einem zu beduftendem Raum befindet ( 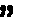 Duftbaum"). Diese Vorrichtung setzt seinen Wirkstoff ständig frei.

Aufgabe der vorliegenden Erfindung war deshalb die effiziente Ausbringung von Flüssigkeiten in Raumluft durch eine technisch einfache, transportfähige und zeitlich regelbare technische Lösung.

### Darstellung der Erfindung und bevorzugter Ausführungsformen

Die oben gestellte Aufgabe konnte mit der vorliegenden Erfindung im Rahmen der Beschreibung und der Ansprüche gelöst werden, indem das folgende Verfahren zur Verfügung gestellt wird. Dieses Verfahren zur effizienten Verdunstung von Flüssigkeiten in einen Luftstrom, ist dadurch gekennzeichnet, daß
(1) ein Teil eines Luftstroms durch einen Behälter mit der zu verdunstenden Flüssigkeit geleitet wird,
(2) im Behälter der Teilluftstrom den in der Gasphase befindlichen Flüssigkeitsanteil aufnimmt,
(3) und der so mit gasförmiger Flüssigkeit angereicherte Teilluftstrom anschließend über eine weitere Behälteröffnung wieder in den Hauptluftstrom gelangt.

Grundvoraussetzung für dieses Verfahren ist das Vorhandensein eines Luftstroms.
Es wurde überraschenderweise gefunden, daß selbst kleinste Luftbewegungen, wie sie beispielsweise beim Öffnen von Raumtüren oder an Klimaanlagen entstehen bzw. vorhanden sind, einen verwendbaren Luftstrom zur Ausübung der Erfindung darstellen.
Bei jeder Luftbewegung bildet sich bei diesem Verfahren ein Teilluftstrom eines Hauptstroms, der zur effizienten Verdunstung der Flüssigkeit als Gas führt. Dies gilt auch für sehr kurzzeitige Luftbewegungen.
Durch Ein- und Ausschalten dieses Luftstroms bzw. mechanisches Verschließen des Zugangs oder Abgangs des Behälters vom und zum Luftstrom kann das Verfahren an-bzw. ausgeschaltet werden. Durch das Verstellen des Winkels der Eingangsöffnung des Teilluftstroms in den Behälter zum Hauptstrom kann der Teilluftstrom stufenlos geregelt werden. Das gleiche gilt für das Verstellen des Winkels der Ausgangsöffnung des Behälters zum Luftstrom.
Auch durch die Größe der Behälteröffnungen läßt sich die Verdunstungsmenge variieren. Ein Luftstrom im Sinne der vorliegenden Erfindung ist jede gerichtete Bewegung von Luft.
Als Teilluftstrom ist der Teil des Luftstroms zu verstehen, der durch den Behälter mit der zu verdunstenden Flüssigkeit geführt wird.
Im Behälter befindet sich die zu verdunstende Flüssigkeit. Die Flüssigkeit steht mit der Gasphase oberhalb der Flüssigkeit im physikochemischen Verdampfungsgleichgewicht.
Der Teilluftstrom, der durch den Behälter strömt, tauscht nun die Luft über der Flüssigkeit aus und verschiebt dieses Gleichgewicht zugunsten der Verdampfung der Flüssigkeit. Die Verdampfungsgeschwindigkeit der Flüssigkeit ist eine Funktion der technischen Dimensionierung des Behälters, der Flüssigkeitsoberfläche, der Luftgeschwindigkeit und der Temperatur von Flüssigkeit und Luftstrom.
Als Behälter ist jede Vorrichtung zu verstehen, in der eine zu verdunstende Flüssigkeit gelagert werden kann, und über der ein Luftvolumen vorhanden ist, der ein Vorbeiströmen des Teilluftstroms ermöglicht, ohne die Flüssigkeit mit in den Hauptstrom mitzureißen.

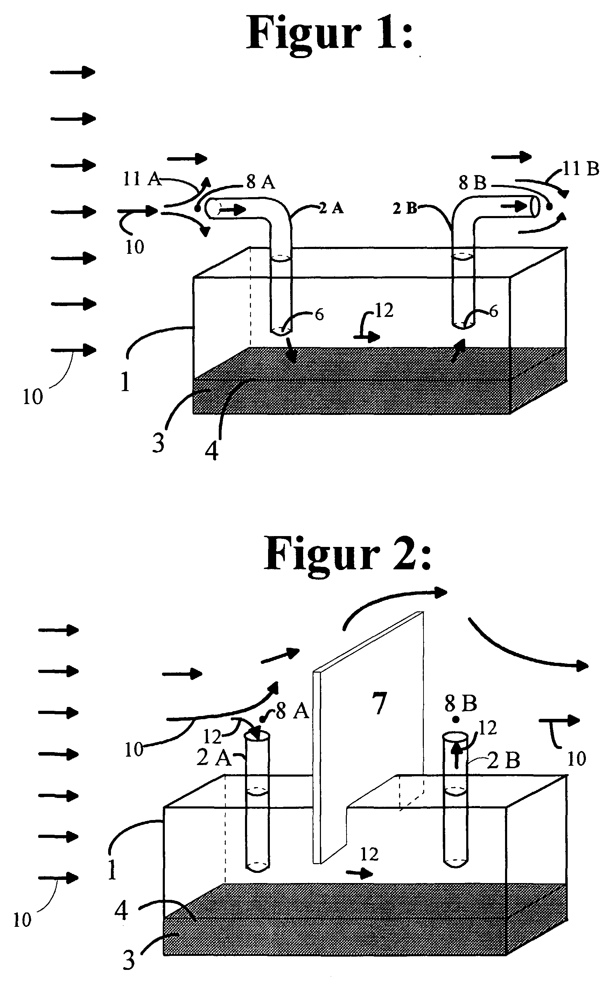

Im allgemeinen ist der Behälter bis auf Ein- und Ausgangsöffnungen gegenüber dem Luftstrom geschlossen. Weitere Öffnungen des Behälters in besonderen Ausführungsformen sind nicht ausgeschlossen.
Die Zuführung des Teilluftstroms kann beliebig technisch realisiert werden, wie beispielsweise über ein oder mehrere zuführende Rohre oder einfach durch eine Öffnung im Behälter. Auch ist es möglich, über die Ausführung der Zuführung die Menge und die Geschwindigkeit des Teilluftstroms bei gegebener Geschwindigkeit des Luftstroms zu bestimmen. In einer Ausführungsform ist das erfindungsgemäße Verfahren dadurch gekennzeichnet, daß mehrere Teilluftströme durch mehrere Zuführungen in den Behälter mit der zu verdunstenden Flüssigkeit gelangen.
Die Abführung des Teilluftstroms nach Beladung im Flüssigkeitsbehälter kann wiederum beliebig technisch realisiert werden, wie beispielsweise über ein oder mehrere abführende Rohre oder einfach durch eine Öffnung im Behälter zum Luftstrom.
Der Teilluftstrom kann durch direktes Umleiten eines Teils des Hauptluftstroms entstehen oder aber auch durch eine Druckdifferenz zwischen der Eingangs- und der Ausgangsöffnung hervorgerufen werden. Diese Druckdifferenz entsteht durch einen Staudruck am Eingang und einen Unterdruck am Ausgang des Behälters. Das Wirkungsprinzip ist schematisch in Figur 1 und 2 wiedergegeben. Die vorliegende Erfindung umfasst in einer bevorzugten Ausführungsform somit erfindungsgemäße Verfahren, die dadurch gekennzeichnet sind, daß der Teilluftstrom durch eine Druckdifferenz zwischen den zu- und abführenden Behälteröffnungen hervorgerufen wird.
Die erwähnte Druckdifferenz kann durch Erhöhung des Luftwiderstands im Hauptstrom zwischen der Behälterzuführung und der Behälterabführung des Teilluftstrom verstärkt werden. Eine mögliche Ausführungsform ist in Figur 2 wiedergegeben. Ein weiteres nichtlimitierendes Beispiel wäre eine Lochblende im Hauptstrom zwischen den jeweiligen Öffnungen.
Die erfindungsgemäßen Verfahren eignen sich besonders gut zur Regelung der auszubringenden Flüssigkeitsmenge. Allein das Abstellen des Luftstroms bzw. des Teilluftstrom ohne Verschluß der Öffnungen vom Luftstrom zum abgeschlossenen Behälter führt dazu, daß keine oder nur eine sehr geringe Menge verdampfte Flüssigkeit den Behälter verläßt.
Die Verwendung von Röhren oder anderer ähnlicher die Teilluft zu- und abführenden Öffnungen zum Behälter erlaubt eine stufenlose Regelung durch Verstellen der Winkel zum Luftstrom. Daher umfasst die Erfindung Verfahren, die dadurch gekennzeichnet sind, daß das Verändern der Anströmwinkel der Behälteröffnungen im Luftstrom eine Regelung der auszubringenden Menge an Flüssigkeit bewirkt.

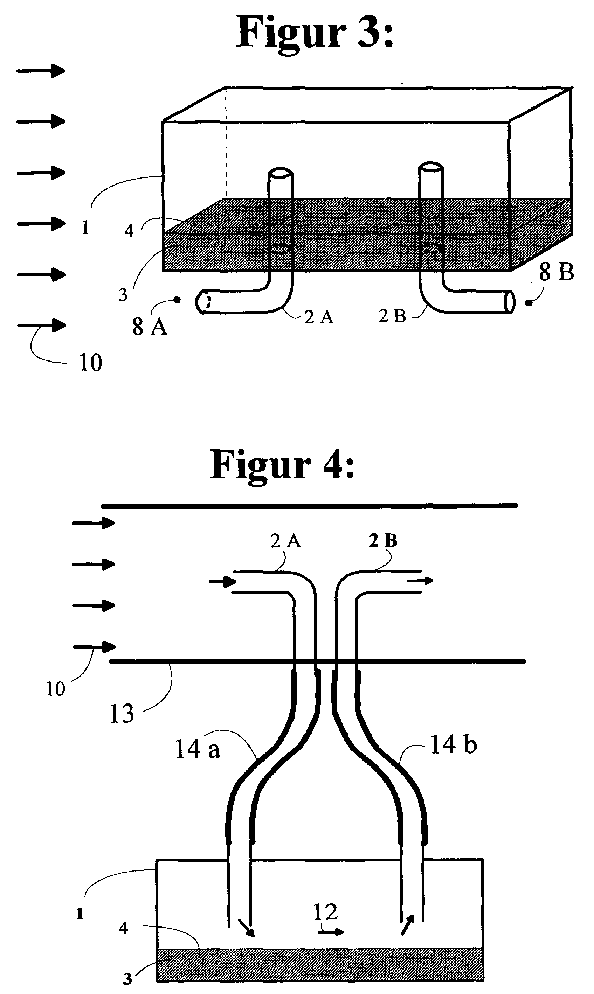

Ein weiteres bevorzugtes Verfahren beinhaltet die Verwendung einer Düse im Behälterinnenraum zur Ausbringung der Flüssigkeit in Tropfenform in den zur Verdunstung zur Verfügung stehenden Behälterinnenraum. Zum einen wird die Verdunstungsoberfläche so erhöht, zum anderen hat dies den Vorteil, daß die Verdunstung so bevorzugt im Mischungsverhältnis der eingesetzten Einzelflüssigkeiten erfolgt. Wenn die Flüssigkeit nicht mechanisch im Gasraum verteilt ausgebracht wird, sondern nur der Verdunstung unterliegt, so werden die unterschiedlichen Partialdrücke der einzelnen Flüssigkeitskomponenten eine zeitabhängige Veränderung der Gesamtflüssigkeits-zusammensetzung bewirken und so Stoffkomponenten mit höheren Partialdrücken akkumulieren. Die Flüssigkeitszufuhr kann sowohl aus dem Inneren des Behälters erfolgen als auch von einer behälterexternen Quelle, wie beispielsweise einem 2. Behälter oder einer Zuleitung.
Auch der Dampfdruck einer Flüssigkeit hat erheblichen Einfluß auf die Dimensionierung der entsprechenden Vorrichtung und Effizienz des Verfahrens. Eine zusätzliche Erwärmung der Flüssigkeit in der erfindungsgemäßen Vorrichtung ist möglich.
Es hat sich herausgestellt, daß das hier beschriebene Verfahren besonders gut zur Verdunstung von flüssigen, natürlichen oder synthetischen Duftstoffen geeignet ist. Neben wässrigen Lösungen werden für die Durchführung des erfindungsgemäßen Verfahrens Flüssigkeiten wie Öle oder wässrige Emulsionen bevorzugt.
Auch biologisch und/oder pharmazeutisch aktive Substanzen in flüssiger Form können erfindungsgemäß raumfüllend und effizient verdunstet werden. Dies findet besonders in der Medizin und der Landwirtschaft Anwendung.
Im ingenieurstechnischen Bereich kann so beispielsweise eine korrosionshemmende Substanz effizient in einen Luftstrom ausgebracht werden.
In einer ganz besonders bevorzugten Ausführungsform betrifft die vorliegende Erfindung Verfahren zur Beduftung von Raumluft mit natürlichen und/oder synthetischen Duftstoffen.
Die Lokalisation des Behälters zum Luftstrom ist nahezu beliebig. Der Behälter kann außerhalb des Luftstroms, also räumlich von diesem getrennt, angeordnet sein (siehe beispielsweise Figur 4 ). Der Behälter kann aber auch direkt im Luftstrom positioniert werden ohne die Funktion des Verfahrens zu beeinträchtigen ( siehe beispielsweise Figuren 1, 2, 3, 5, 6).
Ein weiterer Aspekt der vorliegenden Erfindung betrifft die Vorrichtungen zur Durchführung eines der oben beschriebenen Verfahren. Unter einer solchen Vorrichtung ist generell ein Behälter zu verstehen, der zur Ausübung der oben beschriebenen Verfahren geeignet ist.
Eine solche Vorrichtung kann vielfältig ausgeführt werden. Je nach Bedarf und technischer Vorgabe kann eine solche Vorrichtung über Behälter zu- und abführungen verfügen, die an der Behälteroberfläche enden. In einer bevorzugten Ausführungsform ragen Behälterzu- und abführungen in das Behälterinnere hinein. Diese Ausführungsform verhindert ein unbeabsichtigtes Auslaufen der Flüssigkeit (3) aus dem Behälter (1) . Auch ein beliebiges drehen der Vorrichtung ist dadurch möglich (siehe beispielsweise Figur 3).
Dies ist ein besonderer Vorteil der erfindungsgemäßen Vorichtung zB. beim Beduften eines bewegten Raumes wie beispielsweise in einem Fahrzeug (Auto, Flugzeug).
Besonders bevorzugt sind Vorrichtungen, die dadurch gekennzeichnet sind, daß die Behälterzuführungen aus Röhren ausgebildet sind, die in den Luftstrom hineinragen, wobei die Teilluftzuführung zum Luftstrom hin und die Teilluftabführung vom Luftstrom weg geöffnet ist.
Ganz besonders bevorzugt sind Vorrichtungen, die dadurch gekennzeichnet sind, daß der Behälter eine zusätzliche verschließbare Öffnung zum Befüllen mit Flüssigkeit aufweist, die optional mit einer Verschlußkappe verschließbar ist.
Eine zusätzliche Bewegung der Flüssigkeit innerhalb der Vorrichtung kann deren Oberfläche erhöhen und so die Überführung der Flüssigkeit in die Gasphase verstärken.
Desweiteren sind Vorrichtungen bevorzugt, in deren Behälterinnneren eine Düse zur Ausbringung der Flüssigkeit in das Behältervolumen angeordnet ist. Die Vorteile einer solchen Vorrichtung sind bereits für die erfindungsgemäßen Verfahren beschrieben worden.
Besonders bevorzugt sind Vorrichtungen, bei denen die Oberfäche der Flüssigkeit innerhalb des Behälters durch geeignete Mittel erhöht wird. Die ist beispielsweise durch die Verwendung kapillaraktiver Trägermaterialien möglich.
Solche kapillaraktiven Träger können nicht nur vorteilhaft die Oberfläche der auszubringenden Flüssigkeit erhöhen, sondern auch gleichzeitig ein ungewolltes Auslaufen der Flüssigkeit aus der Vorrichtung vermeiden. In einer ganz besonders bevorzugten Ausführungsform umfasst die vorliegende Erfindung somit erfindungsgemäße Vorrichtungen, in denen die Flüssigkeit in kapillaraktiven Trägermaterialien vorliegt und so eine Auslaufen der Flüssigkeit verhindert. In einer ganz besonders bevorzugten Ausführungsform finden die erfindungsgemäßen Vorrichtungen Verwendung bei der Beduftung von Sanitärräumen.

### Legenden zu den Figuren

Fig. 1. Schematische Darstellung einer beispielhaften erfindungsgemäßen Vorrichtung
   Figur 1 beschreibt eine Vorrichtung, deren geschlossener Behälter (1) eine auszubringende Flüssigkeit (3) bis zu einer bestimmten Füllhöhe (4) enthält, über die ein Teilluftstrom (12) strömt. Der Teilluftstrom (12) ist Teil des Luftstroms (10) der durch die Zuführung 2A in den Behälter gelangt und durch die Behälteröffnung 2B diesen wieder beladen verläßt. Die Zuführungen reichen aussen in den Luftstrom und innen soweit in den Behälter (6), daß dieser selbst bei dem auf den Kopf stellen nicht auslaufen kann.
   Diese Vorrichtung kann in jeden beliebigen Luftstrom gestellt werden oder mit diesem verbunden werden. Eine Regelung des Teilluftstroms kann bei beweglichen Zuführungen (2A, 2B) durch Veränderung des Winkels zum Luftstrom (10) erfolgen.
   Die Figur zeigt schematisch die Entstehung eines Teilstroms durch Druckdifferenzen zwischen den Ein- und Ausgangsöffnungen. Ein Teil des Hauptluftstromes (10) wird am Einlaß der Behälteröffnung (2A) umgelenkt (11A) es entsteht ein Staudruck (8A). Dies führt zu einem Eindringen des Teilluftstromes (12) in den Behälter (1). Die Größe dieses Teilluftstromes (12) wird durch die Anordnung der Behälteröffnung (2B) erhöht. Diese ist von der Hauptluftströmung (10) weggerichtet. Durch die Umlenkung der Hauptluftströmung (11B) entsteht ein Unterdruck (8B) vor der Behälteröffnung (2B).
Fig. 2. Erhöhung der Druckdifferenz durch Erhöhung der Luftwiderstands
   Figur 2 zeigt schematisch die Realisierung eines verstärkten Teilluftstroms durch Positionierung einer Blende zwischen den Ein- und Ausgangsöffnungen, die in einer Erhöhung der Druckdifferenz vor und nach der Blende resultiert. Der Hauptluftstrom (10) wird von einer Blende (7) gestaut und erzeugt dadurch im Einlaßbereich (8A) vor der Behälteröffnung (2A) einen höheren Druck als auf der Auslaßöffnung (2B). Dadurch kommt es zur Ausbildung eines Teilluftstromes (12) durch den Behälter (1).
Fig. 3. Schematische Darstellung einer Vorrichtung mit Teilluftzufuhr von unten
   Figur 3 zeigt, daß durch drehen des Behälters (1) bei Einhaltung einer maximalen Füllhöhe (4) der Flüssigkeit (3) keine unbeabsichtigte Freisetzung der Flüssigkeit (3) erfolgen kann.
Fig. 4. Schematische Darstellung einer Vorrichtung zum Anschluß an eine externe Luftströmung (z B. Lüftungs- oder Klimaschacht).
   Ein Hauptluftstrom (10) innerhalb eines Rohres (13) erzeugt an den Behälteröffnungen (2A/2B) einen Teilluftstrom (12) (siehe Fig.1/2). Dieser wird mittels Zuführungen (14a/14b) zum Behälter (1) geleitet. Der Wirkstoffbehälter (1) ist von dem Hauptluftstrom (10) örtlich getrennt.

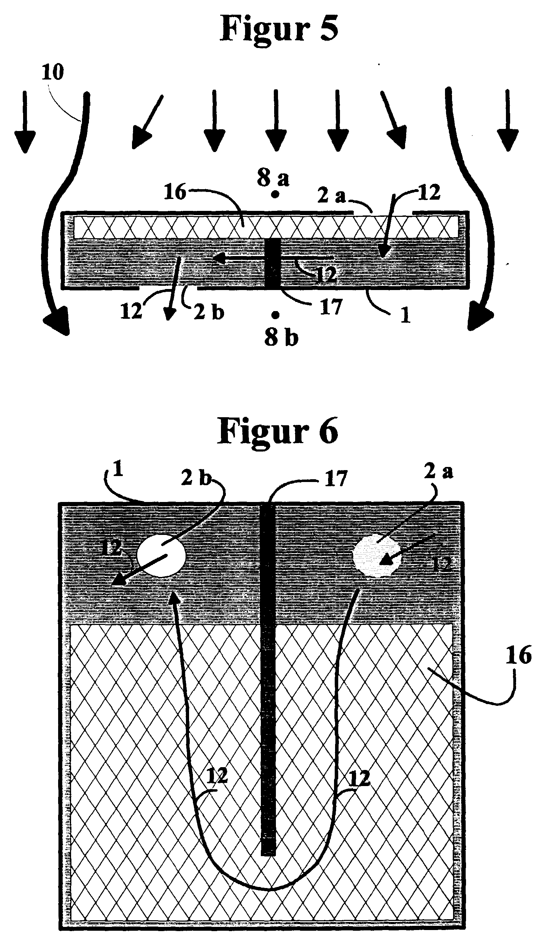

Fig. 5 und 6. Schematische Darstellung eines Beduftungselements mit kapillaraktivem Material als Flüssigkeitsträger.
   Figur 5 und 6 zeigen eine besondere Ausführungsform einer Vorrichtung zur Durchführung der Erfindung. Diese Ausführungsform ist besonders geeignet, um an Türen mit Belüftungsschlitzen, wie beispielsweise Sanitärräumen angewendet zu werden. Der durch die Bewegung der Tür oder durch eine Raumluftabsaugung am Belüftungsschlitz der Tür ausgelöste Luftstrom resultiert in einem Teilluftstrom der erfindungsgemäß über die Öffnung (2a) in die Vorrichtung eintritt. Der Teilluftstrom nimmt die in einem kapillaraktiven Material (16) gelagerte Duftstofflüssigkeit (3) auf und verläßt die Vorrichtung über den Luftaustritt 2b. Eine optionale Trennwand (17) verlängert die Durchlaufstrecke des Teilluftstroms in der Vorrichtung. Der kapillaraktive Flüssigkeitsträger (16) kann die gesammte auszubringende Flüssigkeit enthalten oder zusätzlich mit einem Reservoir verbunden sein.
   Figur 5 zeigt das Beduftungselement (1) von oben, in einem von hinten anströmenden Hauptluftstrom (10). Es zeigt sich die geringe Dicke des Bedufterelementes , welche bei typisch ca. 0,5 bis 2cm liegt.
   Figur 6 zeigt den Duftstoffbehälter von vorne. Eingezeichnet ist der ungefähre Verlauf der Teilluftströmung (12). Der hintere Lufteintritt 2a ist normalerweise nicht zu sehen und wird schraffiert dargestellt. Höhe und Breite des Bedufterelementes betragen jeweils ca.5 bis 15 cm. Die Größe der Behälterüffnungen 2a und 2b sind beliebig den gewünschten Duftstoff emissionsraten anzupassen.

## Patentansprüche

1. Verfahren zur Verdunstung von Flüssigkeiten in einen Luftstrom, das dadurch gekennzeichnet ist, daß
(1) ein Teil eines Luftstroms durch einen Behälter mit der zu verdunstenden Flüssigkeit geleitet wird,
(2) im Behälter der Teilluftstrom den in der Gasphase befindlichen Flüssigkeitsanteil aufnimmt,
(3) und der so mit gasförmiger Flüssigkeit angereicherte Teilluftstrom anschließend über eine weitere Behälteröffnung wieder in den Luftstrom gelangt.

2. Verfahren gemäß Anspruch 1, dadurch gekennzeichnet, daß mehrere Teilluftströme durch mehrere Zuführungen in den Behälter mit der zu verdunstenden Flüssigkeit gelangen.

3. Verfahren gemäß Anspruch 1 oder 2, dadurch gekennzeichnet, daß der mit Flüssigkeit angereicherte Teilluftstrom über mehrere Behälteröffnungen den Behälter verläßt.

4. Verfahren gemäß einem der Ansprüche 1 bis 3, dadurch gekennzeichnet, daß der Teilluftstrom durch eine Druckdifferenz zwischen den zu- und abführenden Behälteröffnungen hervorgerufen wird.

5. Verfahren gemäß Anspruch 4, dadurch gekennzeichnet, daß die Druckdifferenz durch Erhöhung der Luftwiderstands im Luftstrom zwischen der Behälterzuführung und der Behälterabführung des Teilluftstroms verstärkt wird.

6. Verfahren gemäß einem der Ansprüche 1 bis 5, dadurch gekennzeichnet, daß bei Abstellen des Luftstroms und/oder des Teilluftstroms keine oder nur eine sehr geringe Menge verdampfte Flüssigkeit den Behälter verläßt.

7. Verfahren gemäß einem der Ansprüche 1 bis 6, dadurch gekennzeichnet, daß das Verändern der Anströmwinkel der Behälteröffnungen im Luftstrom eine Regelung der zu verdunstenden Menge an Flüssigkeit bewirkt.

8. Verfahren gemäß einem der Ansprüche 1 bis 7, dadurch gekennzeichnet, daß die zu verdunstende Flüssigkeit mittels einer Düse innerhalb des Behältervolumens ausgebracht wird.

9. Verfahren gemäß einem der vorangegangenen Ansprüche, dadurch gekennzeichnet, daß es sich bei der Flüssigkeit um einen Duftstoff handelt.

10. Verfahren gemäß einem der vorangegangenen Ansprüche, dadurch gekennzeichnet, daß es sich bei der Flüssigkeit um ein Öl handelt.

11. Verfahren gemäß einem der Ansprüche 1 bis 8, dadurch gekennzeichnet, daß es sich bei der Flüssigkeit um eine biologisch und/oder pharmazeutisch aktive Substanz handelt.

12. Verfahren gemäß einem der Ansprüche 1 bis 8 zur Verdunstung von Flüssigkeiten, dadurch gekennzeichnet, daß es sich bei der Flüssigkeit um eine korrosionshemmende Substanz handelt.

13. Verfahren gemäß einem der Ansprüche 1 bis 10 zur Beduftung von Raumluft mit Duftstoffen.

14. Verfahren gemäß einem der Ansprüche 1 bis 12, dadurch gekennzeichnet, daß der Behälter im Luftstrom angeordnet ist.

15. Verfahren gemäß einem der Ansprüche 1 bis 12, dadurch gekennzeichnet, daß der Behälter außerhalb des Luftstroms angeordnet ist.

16. Vorrichtung zur Durchführung eines Verfahrens gemäß einem der Ansprüche 1 bis 15.

17. Vorrichtung gemäß Anspruch 16, dadurch gekennzeichnet, daß die Behälterzu- und abführungen an der Behälteroberfläche enden.

18. Vorrichtung gemäß Anspruch 16, dadurch gekennzeichnet, daß die Behälterzu- und abführungen in das Behälterinnere hineinragen.

19. Vorrichtung gemäß einem der Ansprüche 16 bis 18, dadurch gekennzeichnet, daß die Behälterzuführungen aus Röhren ausgebildet sind, die in den Luftstrom hineinragen, wobei die Teilluftzuführung zum Luftstrom hin und die Teilluftabführung vom Luftstrom weg geöffnet ist.

20. Vorrichtung gemäß einem der Ansprüche 16 bis 19, dadurch gekennzeichnet, daß der Behälter eine zusätzliche verschließbare Öffnung zum Befüllen mit Flüssigkeit aufweist, die optional mit einer Verschlußkappe verschließbar ist.

21. Vorrichtung gemäß einem der Ansprüche 16 bis 20, dadurch gekennzeichnet, daß die Behälterzu- und abführungen zu entfernen sind und die daraus resultierenden Behälteröffnungen mit einer Verschlußkappe verschließbar sind.

22. Vorrichtung gemäß einem der Ansprüche 16 bis 21, dadurch gekennzeichnet, daß die Oberfläche der Flüssigkeit innerhalb des Behälters durch dafür geeignete Mittel erhöht wird.

23. Vorrichtung gemäß einem der Ansprüche 16 bis 22, dadurch gekennzeichnet, daß die Verdunstungsoberfläche in dem Behälter durch Bewegung der Flüssigkeit erhöht wird.

24. Vorrichtung gemäß einem der Ansprüche 16 bis 23, dadurch gekennzeichnet, daß im Behälterinneren eine Düse zur Ausbringung der Flüssigkeit in das Behältervolumen angeordnet ist.

25. Vorrichtungen gemäß einem der vorangegangen Ansprüche, dadurch gekennzeichnet, daß die Flüssigkeit in kapillaraktiven Trägermaterialien vorliegt und so ein mögliches Auslaufen der Flüssigkeit verhindert wird.

26. Verwendung einer Vorrichtung gemäß einem der Ansprüche 1 bis 23 zur Beduftung von Sanitärräumen.
